(19) Europäisches Patentamt
European Patent Office
Office européen des brevets

(11) EP 3 230 342 B2

(12) # NEUE EUROPÄISCHE PATENTSCHRIFT
Nach dem Einspruchsverfahren

(45) Veröffentlichungstag und Bekanntmachung des Hinweises auf die Entscheidung über den Einspruch:
**01.04.2026 Patentblatt 2026/14**

(45) Hinweis auf die Patenterteilung:
**03.07.2019 Patentblatt 2019/27**

(21) Anmeldenummer: **15801870.5**

(22) Anmeldetag: **01.12.2015**

(51) Internationale Patentklassifikation (IPC):
***C08G 65/325*** (2006.01)

(52) Gemeinsame Patentklassifikation (CPC):
**C08G 65/2621; C08G 65/2648; C08G 65/2696;**
Y02P 20/582

(86) Internationale Anmeldenummer:
**PCT/EP2015/078126**

(87) Internationale Veröffentlichungsnummer:
**WO 2016/091643 (16.06.2016 Gazette 2016/24)**

(54) **VERFAHREN ZUR HERSTELLUNG EINES POLYETHERAMINS**

METHOD OF PRODUCING A POLYETHERAMINE

PROCÉDÉ DE PRÉPARATION D'UNE POLYÉTHERAMINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.12.2014 EP 14197758**

(43) Veröffentlichungstag der Anmeldung:
**18.10.2017 Patentblatt 2017/42**

(73) Patentinhaber: **BASF SE**
**67056 Ludwigshafen (DE)**

(72) Erfinder:
- **WIGBERS, Christof Wilhelm**
  **68167 Mannheim (DE)**
- **MAEGERLEIN, Wolfgang**
  **67117 Limburgerhof (DE)**
- **KRUG, Thomas**
  **67550 Worms (DE)**
- **MELDER, Johann-Peter**
  **67459 Boehl-Iggelheim (DE)**

(74) Vertreter: **BASF IP Association**
**BASF SE**
**GBI - Z078**
**67056 Ludwigshafen (DE)**

(56) Entgegenhaltungen:
**EP-A1- 0 696 572 EP-A2- 1 028 138**



Processed by Luminess, 75001 PARIS (FR)

## Beschreibung

Die vorliegende Erfindung betrifft ein Verfahren zur Herstellung eines Polyetheramins durch Umsetzung eines Polyetheralkohols, der zuvor in Gegenwart einer basischen Kaliumverbindung als Katalysator synthetisiert wurde, mit Ammoniak in Gegenwart von Wasserstoff und eines Katalysators in einem Reaktor oder mehreren Reaktoren.

Die Verfahrensprodukte finden unter anderem Verwendung in Polyurethan-, Polyharnstoff- und Epoxy-Anwendungen. Sie werden beispielsweise zur Härtung von Epoxidharzen, z.B. bei der Herstellung von Rotorblättern für Windkraftanlagen, eingesetzt, sowie bei der Herstellung von Beschichtungen, Kleb- und Haftstoffen. Darüber hinaus finden sie Verwendung in der Erdölgewinnung und in der Bauindustrie.

WO 2011/067199 A1 (BASF SE) betrifft bestimmte aluminiumoxid-, kupfer-, nickel-, kobalt- und zinnhaltige Katalysatoren und ihre Verwendung in Verfahren zur Herstellung eines Amins aus einem primären oder sekundären Alkohol, Aldehyd und/oder Keton. Die Herstellung von Polyetheraminen aus entsprechenden Polyetheralkoholen und Ammoniak wird auf Seite 26, Zeilen 1 bis 5, allgemein erwähnt.

EP 696 572 A1 (BASF AG) betrifft aminierende Hydrierungen unter Verwendung von $ZrO_2$ / CuO / NiO / $MoO_3$ -Katalysatoren. Die Herstellung von Polyetheraminen aus entsprechenden Polyetheralkoholen und Aminierungsmitteln wie Ammoniak wird allgemein gelehrt und im Beispiel 9 (Seite 11) beschrieben.

WO 09/092724 A1 (BASF SE) lehrt Reaktoren zur Durchführung von Hochdruck-Reaktionen und unter anderem ein Verfahren zur Herstellung Polyetheraminen aus den entsprechenden Polyetheralkoholen und Ammoniak, das in solchen Reaktoren durchgeführt wird.

EP 1 028 138 A2 (BASF Corp.) beschreibt die Herstellung von Polyetheralkoholen aus entsprechenden Alkylenoxiden durch Polymerisation in Gegenwart alkalischer Katalysatoren, wie z.B. Kaliumhydroxid, also einer Kaliumverbindung. Der alkalische Katalysator im Rohprodukt wird mittels einer Carbonsäure neutralisiert, die Abtrennung des entstehenden Salzes, z.B. Kaliumsalzes, wird als zusätzlicher Schritt nicht propagiert (vgl. z.B. Absätze [0002], [0003] und [0004].

In WO 07/096317 A1 (BASF AG) wird beschrieben, dass ein Kalium-Gehalt in Polyetheralkoholen für Schaum-Anwendungen unvorteilhaft ist (vgl. z.B. Absätze [0002] und [0003]). Diese Schrift lehrt nichts zur Herstellung von Polyetheraminen.

US 3,580,952 A (Farbwerke Hoechst AG) betrifft eine Polyetheramin-Herstellung durch Umsetzung von Polypropylenoxid mit Ammoniak und lehrt die Abtrennung von Salzen aus dem Produkt erst nach der Aminierungsreaktion (vgl. z.B. das Abstract).

JP 49 014 158 B und JP 49 014 159 B (beide Mitsui Toatsu Chem., Inc.) beschreiben eine Aminierung von Polyetheralkoholen zu Polyetheraminen, wobei vor der Aminierung keine Abtrennung von KOH aus den Polyetheralkoholen erfolgt.

WO 2011/087793 A1 (Huntsman Petrochemical LLC) betrifft Etheramine und ihre Verwendung als Zwischenprodukte bei der Herstellung von Polymeren. Die Verwendung von alkalischen Katalysatoren bei der Alkoxylierung von mehrwertigen Alkoholen wird genannt (Seite 7, Zeilen 3 - 6) und auch die Möglichkeit, diese Katalysatoren nach der Alkoxylierungsreaktion aus dem Rohprodukt abzutrennen, z.B. durch Vakuumstrippen (Seite 7, Zeilen 6 - 9) oder durch Neutralisation mit Säuren, wie z.B. Oxalsäure, oder durch Behandlung mit Magnesiumsilikat und anschließender Filtration (Seite 7, Zeilen 23 - 25, = erster Satz von [0027]).

Bei der Herstellung von Polyetheralkoholen aus einem ein- oder mehrwertigen Alkohol durch Umsetzung mit einem Alkylenoxid oder mehreren Alkylenoxiden, z.B. durch Umsetzung von Monopropylen- oder Dipropylenglykol mit Propylenoxid, werden oft basische Katalysatoren eingesetzt. Bei den basischen Katalysatoren handelt es sich besonders um Alkalimetallverbindungen, ganz besonders Natrium- oder Kaliumverbindungen, beispielsweise um Alkalimetallalkoholate mit in der Regel 1 bis 4 C-Atomen im Alkoholatrest, wie z.B. Natrium- oder Kaliummethylat, Natrium- oder Kaliumethylat, Natrium- oder Kaliumisopropylat, Natrium- oder Kalium-tert.butylat oder Mischungen hieraus. Bei den basischen Katalysatoren handelt es sich weiterhin besonders um Alkali- oder Erdalkalimetallhydroxide, wie z.B. Natrium-, Kalium-, Calcium- oder Bariumhydroxid.

Am meisten bevorzugt wird Kaliumhydroxid als basischer Katalysator eingesetzt.

Nach der Reaktion wird der basische Katalysator in der Regel entweder durch Zusatz von Adsorbentien, wie z.B. Magnesiumsilicat, und anschließende Filtration entfernt oder er wird zunächst mit Hilfe einer Säure neutralisiert und die entstehenden Salze durch Filtration entfernt, ggf. unter Zuhilfenahme von Adsorbentien, wie z.B. Magnesiumsilicat. Zur Neutralisation werden organische Säuren, wie z.B. Essigsäure, oder anorganische Säuren, wie z.B. Schwefelsäure oder Phosphorsäure, verwendet.

Im Zusammenhang mit der vorliegenden Erfindung sind Polyetheralkohole von Bedeutung, die mit Hilfe von Natrium- oder Kaliumverbindungen, besonders Kaliumverbindungen, als basischen Katalysatoren hergestellt wurden. Nach Entfernung des Natrium- bzw. Kaliumkatalysators, z.B. mit Hilfe einer der o.g. Methoden, verbleibt in der Regel in den hergestellten Polyetheralkoholen ein mehr oder weniger hoher Restgehalt an Natrium- bzw. Kaliumionen.

Es wurde beobachtet, dass im Verlauf der, z.B. kontinuierlichen, Umsetzung eines Polyetheralkohols mit

Ammoniak an einem Katalysator, z.B. einem Festbettkatalysator, besonders einem Aluminiumoxid-, Zirkoniumdioxid- oder Chromoxid-geträgerten Kupferkatalysator, die Aktivität des Aminierungskatalysators stetig abnimmt. Während der Produktion muss daher die Produktionstemperatur mit der Zeit jeweils erhöht werden, um den Aktivitätsverlust des Katalysators auszugleichen und um den spezifikationsgerechten Umsatz zu erzielen (Aminzahlen, Aminierungsgrad). Die nötige Temperaturerhöhung ist häufig mit einer Zunahme an unerwünschten Nebenreaktionen verbunden (z.B. Spaltung der Etherketten unter Bildung von kürzeren aminierten Bruchstücken, die zu unerwünschten Nebenprodukten wie z.B. Dimethylmorpholin reagieren können, oder vermehrte Bildung von sekundären bzw. tertiären Aminen). Sobald die maximal mögliche bzw. sinnvolle Reaktionstemperatur erreicht ist, kann der Katalysator durch eine Spülung mit einem geeigneten Lösungsmittel, wie z.B. Wasser und/oder Ammoniak, von Ablagerungen freigewaschen werden, wenn er weiter verwendet werden soll. Im Anschluss an eine solche Spülung, z.B. Wasserspülung, erreicht der Katalysator wieder eine wesentlich höhere Aktivität oder sogar die ursprüngliche Aktivität und die Aminzahl-Spezifikation des Polyetheramins wird erneut bei geringeren Temperaturen erreicht.

Der vorliegenden Erfindung lag die Aufgabe zugrunde, die Wirtschaftlichkeit bisheriger Verfahren zur Herstellung von Polyetheraminen aus Polyetheralkoholen, die in Gegenwart einer basischen Kaliumverbindung als Katalysator hergestellt wurden, zu verbessern und einem Nachteil oder mehreren Nachteilen des Stands der Technik abzuhelfen. Es sollten Maßnahmen gefunden werden, die technisch in einfacher Weise durchzuführen sind und die es erlauben, das Verfahren mit hohem Umsatz, hoher Ausbeute, Raum-Zeit-Ausbeuten (RZA), Selektivität bei bevorzugt gleichzeitig hoher mechanischer Stabilität des Katalysators, z.B. Katalysatorformkörpers, durchzuführen.

Es wurde gefunden, dass durch die Vermeidung, zumindest Reduzierung, von Kaliumionen im eingesetzten Polyetheralkohol die Katalysatoraktivität länger aufrechterhalten werden kann. Natriumionen und Kaliumionen wirken in der, z.B. kontinuierlichen, Aminierung des Polyols als Katalysatorgift (vermutlich durch ihre Ablagerung auf dem Katalysator). Zudem können abgelagerte Natrium- und Kaliumverbindungen, z.B. durch eine Wasser- und/oder Ammoniakspülung, aus dem Reaktor bzw. den Reaktoren, in dem/denen sich der Katalysator befindet, entfernt werden und so die Aktivität des Katalysators wiederhergestellt werden. Dies gelingt umso schneller (Spülzeit) und katalysatorschonender, je mehr in den eingesetzten Polyetheralkoholen der Gehalt an Natrium- und Kaliumionen reduziert ist.

Demgemäß wurde ein Verfahren zur Herstellung eines Polyetheramins durch Umsetzung eines Polyetheralkohols, der zuvor in Gegenwart einer basischen Kaliumverbindung als Katalysator synthetisiert wurde, mit Ammoniak in Gegenwart von Wasserstoff und eines Katalysators in einem Reaktor oder mehreren Reaktoren gefunden, welches dadurch gekennzeichnet ist, dass der eingesetzte Polyetheralkohol einen Gehalt an Kaliumionen von < 10 Gew.-ppm aufweist, wobei als Poletheralkohole Diole der allgemeinen Formel II

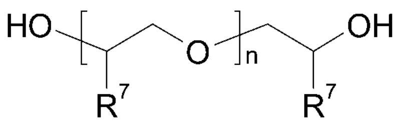

(II)

eingesetzt werden, n jeweils eine ganze Zahl zwischen 1 und 50 und $R^7$ lineares $C_1$-Alkyl bedeuten.

Durch die Vermeidung häufiger Spülzyklen des Katalysators bzw. durch die Verlängerung der Produktionszeiten zwischen zwei Spülungen wird ein Kapazitätsgewinn erreicht. Zudem kann durch die länger andauernde hohe Aktivität des Katalysators bei geringeren Temperaturen produziert werden, wodurch die Selektivität gesteigert wird (d.h. Nebenreaktionen vermieden werden) und die Reaktionstemperatur weiter von sicherheitstechnisch maximal zulässigen Temperaturen entfernt ist (bei zu hoher Temperatur sind gegebenenfalls Durchgehreaktionen möglich). Alternativ kann bei einer bestimmten Temperatur durch das erfindungsgemäße Verfahren eine höhere Katalysatorbelastung (kg Polyetheralkohol / ($I_{Kat.}$ • h)) gefahren werden.

Bei der basischen Kaliumverbindung handelt es sich z.B. um Kaliumhydroxid oder Kaliumalkoholate, wie z.B. Kaliummethylat, -ethylat, -isopropylat oder-tert.-butylat, insbesondere um Kaliumhydroxid.

Der eingesetzte Polyetheralkohol weist einen Gehalt an Kaliumionen von < Gew.-ppm, ganz besonders bevorzugt von < 8 Gew.-ppm, z.B. im Bereich von 2 bis 7 Gew.-ppm, auf (jeweils berechnet au 100%ig reinen Polyetheralkohol).

Ein solcher Gehalt an Kaliumionen im Polyetheralkohol kann durch Maßnahmen erzielt werden, wie sie dem Fachmann bekannt sind, z.B. aus EP 1 028 138 A2 (BASF Corp.), dort besonders Absatz [0002], oder aus WO 2011/087793 A1 (Huntsman Petrochemical LLC), dort besonders Seite 7, Zeilen 6 - 9 und 23 - 25. Bevorzugte Methoden zur Einstellung eines Gehalts an Kaliumionen von ≤ 20 Gew.-ppm, bevorzugt im Bereich von 0 bis < 20 Gew.-ppm, z.B. im Bereich von 2 bis 18 Gew.-ppm, sind:

- Vakuumstrippen des Polyetheralkohols, wobei der Polyetheralkohol über Kopf von der basischen Kalium- bzw. Natriumverbindung abgetrennt wird.

- Behandlung mit einem Magnesiumsilikat, z.B. Ambosol®, in Gegenwart einer kleinen Menge an Wasser (z.B. 1 Gew.-% $H_2O$ bezogen auf den reinen Polyetheralkohol) und anschließende Filtration, wobei die betreffenden, entstandenen Kalium- bzw. Natriumsalze im Filterkuchen verbleiben. Siehe z.B.: http://www.pqcorp.com/pc/EMEA/Markets/Polyol-Purification.
- Behandlung mit einem gängigen Ionenaustauscher für Kationen.
- Neutralisierung der basischen Kalium- bzw. Natriumverbindung mit einer Säure, insb. einer anorganischen Säure, besonders Phosphorsäure, wobei das Kalium bzw. Natrium in Form schwerlöslicher Salze gefällt wird, und anschließende Filtration, wobei die betreffenden Kalium- bzw. Natriumsalze im Filterkuchen verbleiben.
- Neutralisierung der basischen Kalium- bzw. Natriumverbindung mit einer Säure, wie z.B. Essigsäure, wobei leicht lösliche bzw. teilweise lösliche Kalium- bzw. Natriumsalze entstehen, und Behandlung mit einem Magnesiumsilicat, z.B. Ambosol®, und anschließende Filtration, wobei die betreffenden Kalium- bzw. Natriumsalze im Filterkuchen verbleiben.

Das erfindungsgemäße Verfahren zur Herstellung eines Polyetheramins wird bevorzugt an einem heterogenen Katalysator durchgeführt.
Ist der Katalysator als Festbett angeordnet, kann es für die Selektivität der Reaktion vorteilhaft sein, den Katalysator, z.B. die Katalysatorformkörper, im Reaktor bzw. in den Reaktoren mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Der Heterogenkatalysator kann entweder in Form einer Suspension oder eines Festbettes vorliegen.
Im Falle einer Suspensions-katalysierten Aminierung kann das Verfahren beispielsweise in einem oder mehreren gerührten Reaktor/en, in einem oder mehreren Blasensäulenreaktor/en oder in einem oder mehreren Strahlschlaufenreaktor/en durchgeführt werden.
Bevorzugt ist die Durchführung der Aminierung der Polyetheralkohole in Festbettreaktoren und besonders bevorzugt in Schachtreaktoren und Rohrbündelreaktoren.
Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Die Reaktoren können jeweils als einzelner Reaktor, als Serie von einzelnen Reaktoren und/oder in Form von zwei oder mehr parallelen Reaktoren eingesetzt werden.
Optional kann bei einer seriellen Reaktorverschaltung eine Zwischeneinspeisung von Feed (enthaltend den Polyetheralkohol und/oder Ammoniak und/oder $H_2$) und/oder Kreisgas und/oder Frischgas und/oder Reaktoraustrag aus einem nachgeschalteten Reaktor vorgesehen werden. Bei einer seriellen Reaktorverschaltung können optional ein oder mehrere Wärmetauscher zwischen die Reaktoren geschalten sein, um die gewünschte Temperatur einzustellen.

Der spezielle Reaktoraufbau und die Durchführung der Reaktion können in Abhängigkeit von dem speziellen umzusetzenden Polyetheralkohol, den erforderlichen Reaktionszeiten und der Zusammensetzung des eingesetzten Katalysators variieren.

Die Fließrichtung der Reaktanden (Polyetheralkohol, Ammoniak, ggf. Wasserstoff, ggf. rückgeführte Gase und/oder Flüssigkeiten) in Festbettreaktoren ist in der Regel von oben nach unten (Rieselfahrweise) bzw. von unten nach oben (Sumpffahrweise).

Die Reaktion kann kontinuierlich oder diskontinuierlich durchgeführt werden. Bevorzugt ist die kontinuierliche Fahrweise. Bei der kontinuierlichen Fahrweise ist der Katalysator bevorzugt als Festbett im Reaktor bzw. in den Reaktoren angeordnet.

Das Verfahren kann in isothermer oder adiabater Weise durchgeführt werden. Eine isotherme Betriebsweise kann beispielsweise dadurch erreicht werden, dass die bei der Aminierung der Polyetheralkohole im Reaktor bzw. in den Reaktoren freiwerdende Reaktionsenthalpie durch geeignete innen- oder außenliegende Kühlaggregate abgeführt wird.
Im Wesentlichen isotherme Bedingungen im Sinne der vorliegenden Erfindung bedeutet, dass sich die Temperatur im Inneren des Rohres um maximal 6 K, vorzugsweise um maximal 3 K, erhöht. Die Temperaturdifferenz wird aus der Temperatur am Reaktorausgang und derjenigen am Reaktoreingang ermittelt.
Eine isotherme Farhrweise kann besonders bevorzugt in einem oder mehreren Rohrbündelreaktor/en erfolgen.
Dabei können Rohrbündelreaktoren verwendet werden, wie sie in WO 09/092724 A1 (BASF SE) beschrieben sind. Es ist bevorzugt, dass sich der Katalysator und das Reaktionsmedium innerhalb der Rohre befinden und das Kühlmedium sich im Mantelraum um die Rohre herum befindet. In einer besonders bevorzugten Ausführungsform wird siedendes Wasser als Kühlmedium verwendet.
Je nach Betriebsbedingungen des/der Rohrbündelreaktor/s/en kann man von einer rein isothermen Betriebsweise (mit o.g. Temperaturerhöhung über die Katalysatorschüttung) in Richtung einer adiabaten Betriebsweise gehen, wobei die Temperaturerhöhung in den Rohren beispielsweise im Falle von Polyetheramin D230 (s.u.) dann bis zu 15 K betragen kann.

Bei einer adiabaten Betriebsweise wird die freiwerdende Reaktionsenthalpie nicht abgeführt, sondern verbleibt

im Reaktionsgemisch. Führt man die Reaktion in einem bzw. mehreren Festbettreaktor/en durch, so führt eine adiabate Verfahrenweise zu einer Temperaturerhöhung des Reaktionsgemischs von bis zu 30 °C oder mehr beim Durchgang durch den Reaktor, je nach eingestellten Reaktionsbedingungen, wie z.B. Ammoniak/Polyetheralkohol-Molverhältnis (siehe unten), Druck und ggf. Kreisgasmenge. Zur Kontrolle und Überwachung der Temperatur können mehrere Messstellen im Reaktor bzw. in den Reaktoren angebracht sein.

Der adiabate Temperaturanstieg lässt sich auch dadurch begrenzen, dass man einen Teil des flüssigen Rohaustrags aus der Aminierung wieder an den Reaktoreingang zurückführt und gemeinsam mit dem Polyetheralkohol und dem Ammoniak durch den Reaktor leitet.

Die Katalysatorbelastung bei kontinuierlicher Fahrweise liegt besonders im Bereich von 0,01 bis 10, vorzugsweise im Bereich von 0,1 bis 2,0, besonders bevorzugt im Bereich von 0,15 bis 1,0 kg Polyetheralkohol pro Liter Katalysator (Schüttvolumen) und Stunde.

Gegebenenfalls kann eine Verdünnung der Edukte mit einem geeigneten, unpolaren oder bevorzugt polaren Lösungsmittel, wie Tetrahydrofuran, Dioxan oder Ethylenglykoldimethylether, erfolgen.

Das erfindungsgemäße Verfahren zur Aminierung von Polyetheralkoholen wird vorzugsweise bei einer Temperatur im Bereich von 150 bis 240 °C, insbesondere im Bereich von 170 bis 230 °C, weiter besonders im Bereich von 180 bis 220 °C, ganz besonders im Bereich von 190 bis 215 °C, durchgeführt.

Die Umsetzung wird bevorzugt in der Flüssigphase durchgeführt. Dies bedeutet, dass sich der Edukt-Alkohol und das Produkt-Amin unter den Reaktionsbedingungen in flüssiger Form im Reaktor bzw. in den Reaktoren befinden.

Der Reaktionsdruck beträgt vorzugsweise 50 bis 220 bar, weiter bevorzugt 75 bis 200 bar, insbesondere 100 bis 180 bar, weiter besonders 110 bis 160 bar.

Der Druck im Reaktionsgefäß, welcher sich aus der Summe der Partialdrücke von Ammoniak, des Polyetheralkohols, der gebildeten Reaktionsprodukte sowie ggf. eines mitverwendeten Lösungsmittels und/oder rückgeführter gas- oder flüssiger Komponenten bei den angegebenen Temperaturen ergibt, wird zweckmäßigerweise durch das Zuführen des Wasserstoffs auf den gewünschten Reaktionsdruck erhöht.

Ammoniak wird bevorzugt in einem Molverhältnis im Bereich von 1,5- bis 500 pro Mol alkoholischer Hydroxylgruppe im Polyetheralkohol eingesetzt. Besonders liegt dieses Molverhältnis im Bereich von 3 bis 150, weiter besonders im Bereich von 5 bis 120.

Der gewählte Wert bezüglich dieses Molverhältnisses kann von der Art des eingesetzten Polyetheralkohols abhängen. So liegt beispielsweise bei der Aminierung von Polypropylenglykol P230 (einem Gemisch aus Molekülen der Formel IIa mit einer mittleren Molmasse im Bereich von 210 bis 250 g/mol, besonders von 230 g/mol) dieses Molverhältnis bevorzugt im Bereich von 5 bis 20. Bei der Aminierung von Polypropylenglykol P2000 (einem Gemisch aus Molekülen der Formel IIb mit einer mittleren Molmasse im Bereich von 1900 bis 2100 g/mol, besonders von 2000 g/mol) liegt dieses Molverhältnis bevorzugt im Bereich von 75 bis 120.

Die Aminierung der Polyetheralkohole wird in Gegenwart von Wasserstoff durchgeführt.

Nach dem Durchtritt durch den Reaktor bzw. die Reaktoren wird das heiße Reaktionsgemisch in der Regel mit Hilfe eines Wärmetauschers oder auch mehrerer Wärmetauscher abgekühlt. Die Wärmetauscher können beispielsweise mit Luft oder mit Wasser als Kühlmedium betrieben werden. Als Kühlmedium kann auch das kalte Zulaufgemisch zum Reaktor dienen.

Um gasförmige von flüssigen Komponenten zu trennen, wird das Reaktionsgemisch zweckmä-ßigerweise in einen oder mehrere Abscheidebehälter geleitet, die in der Regel bei unterschiedlichen Drucken betrieben werden. Die gasförmigen Komponenten können entweder als Kreisgas zurück zum Reaktoreingang geleitet werden oder als Abgas aus dem Prozess ausgeschleust werden.

Es kann zwischen einer Fahrweise unterschieden werden, bei der die Gasphase im geraden Durchgang durch den bzw. die Reaktor/en geführt wird und nach dem Durchgang durch den bzw. die Reaktor/en als Abgas ausgeschleust wird (= Frischgasfahrweise) und einer Fahrweise, bei der die Gasphase vollständig oder teilweise nach Durchgang durch den bzw. die Reaktor/en wieder vor den Reaktor zurückgeführt wird (= Kreisgasfahrweise). Für die Kreisgasfahrweise kann man technisch einen Kreisgaskompressor einsetzen, der die Gasphase nach dem Durchgang durch den Reaktor und Abtrennung von der Flüssigphase wieder verdichtet und sie zum Reaktoreingang zurückführt.

Bei einer Frischgasfahrweise fährt man bevorzugt Wasserstoff in einer Menge von 1 bis 200 $Nm^3$ / [$m^3$ Katalysator (Schüttvolumen) • h], bevorzugt 5 bis 100 $Nm^3$ / [$m^3$ Katalysator (Schüttvolumen) • h], in den Reaktor. Da diese Wasserstoffmenge als Abgas verloren geht, ist die gewählte Menge u.a. eine wirtschaftliche Abwägung.

Bei einer Kreisgasfahrweise liegt die Kreisgasmenge bevorzugt im Bereich von 50 bis 1000 $Nm^3$ / [$m^3$ Katalysator (Schüttvolumen) • h], insbesondere im Bereich von 60 bis 300 $Nm^3$ / [$m^3$ Katalysator (Schüttvolumen) • h]. Das Kreisgas enthält bevorzugt mindestens 10, besonders 50 bis 100 und ganz besonders 60 bis 95, Vol.% $H_2$. Den Rest bildet überwiegend Ammoniak. Die Zusammensetzung des Kreisgases wird auch durch die gewählte Temperatur im o.g. Abscheidebehälter bestimmt, die z.B. im Bereich von 0 °C bis 60 °C, bevorzugt zwischen 20 und 40 °C, liegen kann.

[Normkubikmeter = $Nm^3$ = auf Normalbedingungen (20 °C, 1 bar abs.) umgerechnetes Volumen].
Katalysatorvolumen-Angaben beziehen sich immer auf das Schüttvolumen.

Üblicherweise werden bei der Aminierung der Polyetheralkohole Aminierungsgrade im Bereich von 60 bis 100 % erreicht, bevorzugt im Bereich von 80 bis 95 %.

Es ist zweckmäßig, die Reaktanden bereits vor der Zuführung in das Reaktionsgefäß zu erwärmen, und zwar bevorzugt auf die Reaktionstemperatur.

Dementsprechend sieht ein mögliches Verfahrenskonzept mit kontinuierlichem Betrieb vor, dass die Ausgangsstoffe (Polyetheralkohol, Ammoniak und Wasserstoff) zusammen mit eventuell rückgeführten Gas- und/oder Flüssigströmen getrennt oder zusammen durch einen oder mehrere Vorheizer gefahren werden, um die gewünschte Reaktoreintrittstemperatur einzustellen. Der oder die Vorheizer werden dabei üblicherweise mit Dampf beheizt. Als Heizmedium kann jedoch auch heißer Reaktionsaustrag dienen.

Die Ausgangsstoffe sowie eventuell rückgeführte Gas- und/oder Flüssigströme können vermischt werden, bevor sie in den Reaktor geleitet werden, oder sie können getrennt auf den Reaktoreingang geführt werden.

Der flüssige Reaktionsaustrag wird in der Regel von Katalysatorresten befreit, z.B. durch Filtration, Zyklone etc.

Der flüssige Reaktionsaustrag wird zweckmäßigerweise in einem Destillationsteil aufgearbeitet.

Üblicherweise befindet sich im flüssigen Reaktionsaustrag noch überschüssiger Ammoniak. Der Ammoniak wird im Besonderen durch Destillation abgetrennt und bevorzugt in die Umsetzung zurückgeführt.

Das im Zuge der Umsetzung gebildete Reaktionswasser, jeweils ein Mol pro Mol umgesetzte Alkoholgruppe, wirkt sich im Allgemeinen auf den Umsetzungsgrad, die Reaktionsgeschwindigkeit, die Selektivität und die Katalysatorstandzeit nicht störend aus und wird deshalb zweckmä-ßigerweise erst bei der destillativen Aufarbeitung des Reaktionsproduktes aus diesem entfernt.

Eine Aufarbeitung des Produkts der Umsetzung ist bevorzugt wie folgt ausgestaltet:

Da die Polyetheramine üblicherweise hochsiedende Produkte sind, werden sie im Destillationsteil des Verfahrens bevorzugt als Sumpfprodukte gewonnen. Wasser (siehe oben) sowie andere Leichtsieder werden über Kopf abdestilliert.

Um hohe Sumpftemperaturen zu vermeiden, kann die destillative Entfernung des Wassers und ggf. anderer Leichtsieder unter reduziertem Druck (Vakuum) durchgeführt werden.

Falls das Polyetheramin (PEA) als Sumpfprodukt gewonnen wird, kann eine zu lange thermische Beanspruchung des PEAs dadurch vermieden werden, dass die Verweilzeit durch die Größe des Sumpfbereiches der Destillationskolonne und/oder den Durchsatz begrenzt wird. Bevorzugt liegt eine solche Verweilzeit im Bereich von 5 bis 60 Minuten.

Bevorzugt ist eine Verfahrensweise, bei der aus dem Reaktionsprodukt der Umsetzung durch Destillation

(i) zunächst ggf. unumgesetzter Ammoniak über Kopf abgetrennt und bevorzugt in das Verfahren zurückgeführt wird,
(ii) Wasser über Kopf abgetrennt wird,
(iii) ggf. vorhandene Nebenprodukte mit einem niedrigeren Siedepunkt als dem des Verfahrensprodukts über Kopf, ggf. zusammen mit noch vorhandenem Wasser, abgetrennt werden,
und (iv) das Verfahrensprodukt Polyetheramin über Sumpf abgetrennt wird.

Mit dem erfindungsgemäßen Verfahren bevorzugt herstellbar sind Polyetheramine der folgenden Formel

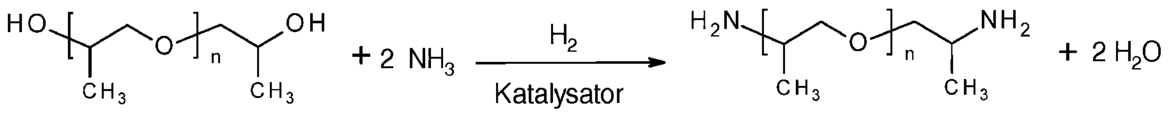


IIa

wobei Polyetheralkohol IIa (Edukt) und Polyetheramin (Produkt) jeweils als Mischung aus Molekülen vorliegen, bei denen n im Mittel im Bereich von 2,3 bis 3,0, besonders im Bereich von 2,5 bis 2,8, liegt und die Molmasse des Polyetheramins im Mittel im Bereich von 210 bis 250 g/mol, besonders im Bereich von 220 bis 240 g/mol, z.B. bei 230 g/mol, liegt.

Weiterhin sind mit dem erfindungsgemäßen Verfahren bevorzugt Polyetheramine der folgenden Formel herstellbar

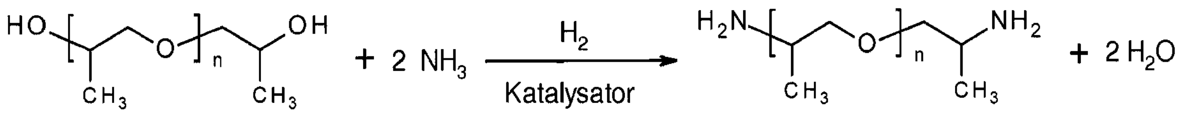


IIb

wobei Polyetheralkohol IIb (Edukt) und Polyetheramin (Produkt) jeweils als Mischung aus Molekülen vorliegen, bei denen n im Mittel im Bereich von 31,5 bis 35,0, besonders im Bereich von 32,3 bis 34,0, liegt und die Molmasse des

Polyetheramins im Mittel im Bereich von 1900 bis 2100 g/mol, besonders im Bereich von 1950 bis 2050 g/mol, z.B. bei 2000 g/mol, liegt.

Der bevorzugt im erfindungsgemäßen Verfahren eingesetzte Katalysator enthält Kupfer und/oder Kobalt und/oder Nickel.

Bevorzugt ist, dass die katalytisch aktive Masse des Katalysators vor dessen Reduktion mit Wasserstoff sauerstoffhaltige Verbindungen des Aluminiums und/oder Zirkoniums und/oder Chroms und sauerstoffhaltige Verbindungen des Kupfers enthält.

Weiter bevorzugt ist, dass die katalytisch aktive Masse des Katalysators vor dessen Reduktion mit Wasserstoff sauerstoffhaltige Verbindungen des Aluminiums und/oder Zirkoniums und/oder Chroms und sauerstoffhaltige Verbindungen des Kupfers und Nickels enthält.

Weiter bevorzugt ist, dass die katalytisch aktive Masse des Katalysators vor dessen Reduktion mit Wasserstoff sauerstoffhaltige Verbindungen des Aluminiums und/oder Zirkoniums und sauerstoffhaltige Verbindungen des Kupfers und Kobalts und Nickels enthält.

In einer besonderen Ausgestaltung enthält die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-%, besonders 0,4 bis 4 Gew.-%, sauerstoffhaltige Verbindungen des Zinns, berechnet als SnO.

Zum Beispiel ist der in WO 2011/067199 A1 (BASF SE) und z.B. auch in WO 2014/009292 A1 oder in PCT/EP2014/059181 offenbarte Katalysator, in dem die katalytisch aktive Masse des Katalysators vor dessen Reduktion mit Wasserstoff im Bereich von

15 bis 80 Gew.-%, besonders 30 bis 70 Gew.-%, weiter besonders 35 bis 65 Gew.-%, sauerstoffhaltige Verbindungen des Aluminiums, berechnet als $Al_2O_3$,

1 bis 20 Gew.-%, besonders 2 bis 18 Gew.-%, weiter besonders 5 bis 15 Gew.-%, sauerstoffhaltige Verbindungen des Kupfers, berechnet als CuO, und

5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Nickels, berechnet als NiO,

5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Kobalts, berechnet als CoO, und

0,2 bis 5,0 Gew.-%, besonders 0,4 bis 4,0 Gew.-%, weiter besonders 0,6 bis 3,0 Gew.-%, weiter besonders 0,7 bis 2,5 Gew.-%, sauerstoffhaltige Verbindungen des Zinns, berechnet als SnO, enthält, vorteilhaft einsetzbar.

In diesem Katalysator beträgt das Molverhältnis von Nickel zu Kupfer bevorzugt größer 1, besonders bevorzugt größer 1,2, weiter besonders bevorzugt liegt es im Bereich von 1,8 bis 8,5. Die BET-Oberfläche (ISO 9277:1995) dieses Katalysators liegt bevorzugt im Bereich von 30 bis 250 $m^2/g$, besonders im Bereich von 90 bis 200 $m^2/g$, weiter besonders im Bereich von 130 bis 190 $m^2/g$, (jeweils vor der Reduktion mit Wasserstoff). Diese Bereiche werden insbesondere durch Kalcinierungstemperaturen bei der Katalysatorherstellung im Bereich von 400 bis 600 °C, besonders 420 bis 550 °C, erzielt.

Insbesondere kann beispielsweise der in WO 2011/067199 A1, Beispiel 5, Seiten 28 und 29, offenbarte Katalysator eingesetzt werden.

Zum Beispiel ist in einer anderen besonderen Ausgestaltung auch der in EP 696 572 A1 (BASF SE) und z.B. auch in PCT/EP2014/059145 offenbarte Katalysator, in dem die katalytisch aktive Masse des Katalysators vor dessen Reduktion mit Wasserstoff im Bereich von

20 bis 85 Gew.-%, bevorzugt 20 bis 65 Gew.-%, besonders bevorzugt 22 bis 40 Gew.-%, sauerstoffhaltige Verbindungen des Zirkoniums, berechnet als $ZrO_2$,

1 bis 30 Gew.-%, besonders bevorzugt 2 bis 25 Gew.-%, sauerstoffhaltige Verbindungen des Kupfers, berechnet als CuO,

14 bis 70 Gew.-%, bevorzugt 15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist, und 0 bis 5 Gew.-%, besonders 0,1 bis 3 Gew.-%, sauerstoffhaltige Verbindungen des Molybdäns, berechnet als $MoO_3$, enthält, vorteilhaft einsetzbar.

Insbesondere beispielsweise der in EP 696 572 A1, Seite 8, offenbarte Katalysator mit der Zusammensetzung 31,5 Gew.-% $ZrO_2$, 50 Gew.-% NiO, 17 Gew.-% CuO und 1,5 Gew.-% $MoO_3$.

(Die Konzentrationsangaben (in Gew.-%) der Komponenten der Katalysatoren beziehen sich jeweils auf die katalytisch aktive Masse des fertigen Katalysators nach dessen letzter ggf. erfolgter Wärmebehandlung und vor dessen Reduktion mit Wasserstoff.

Die katalytisch aktive Masse des Katalysators, nach dessen letzter ggf. erfolgter Wärmebehandlung und vor dessen Reduktion mit Wasserstoff, ist als die Summe der Massen der katalytisch aktiven Bestandteile und der Katalysatorträgermaterialien (Aluminiumoxid bzw. Zirkoniumdioxid) definiert und enthält im Wesentlichen die folgenden Bestandteile: Aluminiumoxid ($Al_2O_3$) bzw. Zirkoniumdioxid ($ZrO_2$), sauerstoffhaltige Verbindungen des Kupfers und Nickels und ggf. des Molybdäns oder Kobalts und Zinns.

Die Summe der o. g. Bestandteile der katalytisch aktiven Masse beträgt üblicherweise 70 bis 100 Gew.-%, bevorzugt 80

bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.)

In einer weiteren besonderen Ausgestaltung sind Katalysatoren bevorzugt, deren Gehalt an Kobalt und/oder Nickel mehr als 90 Gew.-%, besonders mehr als 95 Gew.-%, jeweils bezogen auf das Katalysatorgesamtgewicht ohne ggf. vorhandenes Trägermaterial, beträgt.

Auch sind Katalysatoren bevorzugt, deren Gehalt an Aluminium + Kobalt und/oder Nickel mehr als 80 Gew.-%, besonders mehr als 90 Gew.-%, jeweils bezogen auf das Katalysatorgesamtgewicht ohne ggf. vorhandenes Trägermaterial, beträgt. Zu solchen bevorzugten Katalysatoren gehören Kobaltschwamm- und Nickelschwamm-Katalysatoren, z.B. herstellbar aus Co/Al- bzw. Ni/Al-Legierungen.

Beispielsweise sind Raney®-Kobalt- und Raney®-Nickel-Typen geeignete Katalysatoren, wobei diese Katalysatoren, die Aluminium enthalten, auch mit weiteren Metallen, wie Cr und/oder Mo und/oder Fe und/oder anderen Metallen der Gruppe VIII des Periodensystems (Chemical Abstracts Service group notation), dotiert sein können.

In einer weiteren besonderen Ausgestaltung sind Kobalt-haltige Katalysatoren enthaltend Mangan und Phosphor bevorzugt, besonders die in EP 636 409 A1 und EP 742 045 A1 (beide BASF AG) gelehrten Katalysatoren bevorzugt, deren katalytisch aktive Masse aus 55 bis 98 Gew.-% Kobalt, 0,2 bis 15 Gew.-% Phosphor, 0,2 bis 15 Gew.-% Mangan und 0,05 bis 5 Gew.-% Alkalimetall, jeweils berechnet als Oxid, besteht, welche insbesondere dadurch gekennzeichnet sind, dass man die kalzinierten Katalysatoren bei Endtemperaturen von 200 bis 400°C im Wasserstoffstrom reduziert und anschließend durch Behandlung im Luftstrom bei Endtemperaturen von 20 bis 60°C oberflächlich anoxidiert.

Die Kobaltkatalysatoren weisen eine spezifische Oberfläche (ISO 9277:1995) von ≥ 12 $m^2$/g, besonders 12 bis 500 $m^2$/g, bevorzugt 15 bis 200 $m^2$/g, besonders bevorzugt 18 bis 100 $m^2$/g, und eine Porosität von ≥ 0,16 $cm^3$/g, besonders 0,16 bis 1,00 $cm^3$/g, bevorzugt 0,18 bis 0,80 $cm^3$/g, besonders bevorzugt 0,20 bis 0,40 $cm^3$/g, auf (DIN 66133:1993-06). Die Katalysatoren zeichnen sich ferner dadurch aus, dass im aktivierten Zustand mindestens 85 Gew.-%, also 85 bis 100 Gew.-%, bevorzugt mindestens 95 Gew.-%, also 95 bis 100 Gew.-%, des metallischen Kobalts in hexagonaler Modifikation vorliegt.

Die katalytisch aktive Masse dieser Kobaltkatalysatoren besteht aus 55 bis 98 Gew.-%, bevorzugt 75 bis 95 Gew.-%, besonders bevorzugt 85 bis 95 Gew.-%, Kobalt,

0,2 bis 15 Gew.-%, bevorzugt 0,5 bis 10 Gew.-%, besonders bevorzugt 1 bis 6 %, Phosphor, 0,2 bis 15 Gew.-%, bevorzugt 2 bis 10 Gew.-%, besonders bevorzugt 3 bis 8 Gew.-%, Mangan und 0,05 bis 5 Gew.-%, bevorzugt 0,1 bis 3 Gew.-%, besonders bevorzugt 0,13 bis 1 Gew.-%, Alkalimetall, jeweils berechnet als Oxid ($CoO$, $H_3PO_4$, $MnO_2$, $Alkalimetall_2O$). Als Alkalimetall eignen sich bevorzugt Lithium, Natrium, Kalium und/oder Casium, besonders bevorzugt Natrium und/oder Kalium.

Insbesondere bevorzugt ist der in EP 742 045 A1, Seite 4 oben, offenbarte Katalysator "A".

Zur Herstellung der Polyetheramine gemäß dem erfindungsgemäßen Verfahren Diole der allgemeinen Formel II, eingesetzt.

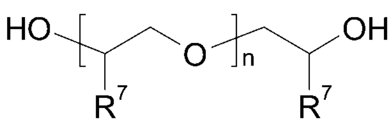

(II)

Darin bedeutet n jeweils eine ganze Zahl zwischen 1 und 50, $R^7$ lineares $C_1$-Alkyl.

Es handelt sich bei dem erfindungsgemäß umzusetzenden Polyetheralkohol um einen sekundären Alkohol und bei dem so hergestellten Polyetheramin um ein primäres Amin.

Alle Druckangaben beziehen sich auf den Absolutdruck.

Alle ppm-Angaben beziehen sich auf die Masse.

Beispiele

1. Herstellung des Katalysators A

Der Katalysator A wurde gemäß Beispiel 5 der WO 2011/067199 A1 (BASF SE) hergestellt. Der so erhaltene Katalysator hatte die Zusammensetzung wie in der folgenden Tabelle I dargestellt.

Tabelle I

| Katalysator *) | Ni | Co | Cu | Sn | BET **) | Träger |
|---|---|---|---|---|---|---|
| | % | % | % | % | $m^2$/g | |

(fortgesetzt)

| Katalysator *) | Ni | Co | Cu | Sn | BET **) | Träger |
|---|---|---|---|---|---|---|
| Katalysator A | 18,6 | 17,3 | 10,6 | 1,1 | 187 | $Al_2O_3$ |
| *) Katalysatorzusammensetzung in Gew.-%; Rest bis zu 100 Gew.-% ist der Träger<br>**) ISO 9277:1995 | | | | | | |

2. Umsetzung von Polyetheralkohol (Pluriol®) P230 mit Ammoniak zu PEA D230 in einem kontinuierlich betriebenen Rohrreaktor

Um eine nachvollziehbare Korrelation zwischen der Kaliumionen-Konzentration im Polyetheralkohol und der Geschwindigkeit der Katalysatordesaktivierung zu erstellen, wurden parallel zwei Versuche an der gleichen Charge des Alkoholaminierungs-Katalysators A (in Form von 1,0 - 1,6 mm Splitt, der aus den reduzierten und passivierten Tabletten hergestellt wurde) durchgeführt, wobei Pluriol® P230 mit einem Kaliumionengehalt von 5 ppm bzw. 10-15 ppm als Zulauf miteinander verglichen wurden.

Beispiel 2a (P230 mit 5 ppm $K^+$)

Ein beheizter Rohrreaktor mit 14 mm Innendurchmesser, einem zentral angebrachten Thermoelement und einem Gesamtvolumen von 89 ml wurde im unteren Teil mit einer Schicht Glaskugeln (15 ml) befüllt, darüber mit 70 ml des reduzierten Aminierungskatalysators A und schließlich der restliche Teil wiederum mit Glaskugeln befüllt. Vor der Reaktion wurde der Katalysator bei max. 280 °C unter Wasserstoff (25 Nl/h) [Nl = Normliter = auf Normalbedingungen (20 °C, 1 bar abs.) umgerechnetes Volumen] bei Normaldruck 12 Stunden aktiviert. Durch den Reaktor wurden von unten nach oben 17,5 g/h Pluriol® P230 mit 5 ppm $K^+$, 28 g/h flüssiger Ammoniak und 8 Nl/h Wasserstoff dosiert. Der Reaktor wurde bei einer Temperatur von 193 °C und einem Gesamtdruck von 120 bar gehalten. Im Anschluss an die Probennahme nach 1145 Stunden wurde die Temperatur auf 203°C erhöht. Im Anschluss an die Probennahme nach 1649 Stunden wurde die Anlage fünf Stunden lang mit 30 g/h Wasser und anschließend fünf Tage lang mit 30 g/h Ammoniak gespült und erneut unter den gleichen Bedingungen wie vor der Spülung angefahren.

Das aus dem Reaktor austretende Gemisch wurde jeweils abgekühlt und auf Normaldruck entspannt. Zu verschiedenen Zeitpunkten wurden Proben vom Reaktionsgemisch genommen und analysiert (vgl. Abbildung 1). Vor Bestimmung der Nass-Analytik wurde regelmäßig der Kaliumionengehalt im Zulauf und im Austrag bestimmt.

Beispiel 2b (P230 mit 10-15 ppm $K^+$), nicht erfindungsgemäß

Die Reaktion wurde analog Beispiel 2a in einer baugleichen parallelen Apparatur durchgeführt, jedoch mit Pluriol® P230 mit 10-15 ppm $K^+$. Nach der Wasser/Ammoniak-Spülung nach der Laufzeit von 1649 h wurde die Anlage unter den gleichen Bedingungen wie vor der Spülung angefahren, jedoch wurde Pluriol® P230 mit einem Kaliumionengehalt von 5 ppm eingesetzt. Es wurde dieselbe Katalysatorcharge verwendet und die Reaktionsbedingungen wurden identisch zu Beispiel 2a gefahren.

Tabelle II:

| Laufzeit h | Beispiel | AZ [mg KOH/g] | AC [mg KOH/g] | tert. AZ [mg KOH/g] | Aminierungsgrad [%] | K+ im Zulauf/ Ablauf [ppm] |
|---|---|---|---|---|---|---|
| 41 | 2a | 464,8 | 497,5 | 0,60 | 93,43 | 5 / 0 |
| | 2b | 464,3 | 500,7 | 0,5 | 92,73 | 10 / 0 |
| 137 | 2a | 460,0 | 497,5 | 0,94 | 92,47 | 5 / 0 |
| | 2b | 462,4 | 500,7 | 0,5 | 92,35 | 10 / 0 |
| 305 | 2a | 442,5 | 501,4 | 0,7 | 88,25 | 5 / 0 |
| | 2b | 436,6 | 504,3 | 0,4 | 86,58 | 14 / 0 |
| 473 | 2a | 432,8 | 503,1 | 0,5 | 86,03 | 5 / 0 |
| | 2b | 417,0 | 505,8 | 0,7 | 82,43 | 15 / 0 |
| 641 | 2a | 414,3 | 498,4 | 0,4 | 83,13 | 5 / 0 |

(fortgesetzt)

| Laufzeit h | Beispiel | AZ [mg KOH/g] | AC [mg KOH/g] | tert. AZ [mg KOH/g] | Aminierungsgrad [%] | K+ im Zulauf/ Ablauf [ppm] |
|---|---|---|---|---|---|---|
| | 2b | 395,6 | 498,9 | 0,4 | 79,29 | 15 / 0 |
| 809 | 2a | 396,3 | 504,0 | 0,8 | 78,63 | 5 / 0 |
| | 2b | 366,8 | 507,0 | 0,8 | 72,35 | 15/0 |
| 977 | 2a | 374,1 | 504,5 | 0,8 | 74,15 | 5 / 0 |
| | 2b | 335,6 | 504,9 | 0,8 | 66,47 | 15 / 0 |
| 1145 | 2a | 342,2 | 502,6 | 0,9 | 68,09 | 5 / 0 |
| | 2b | 304,0 | 503,9 | 0,6 | 60,33 | 15 / 0 |
| 1169 | 2a | 414,0 | 502,6 | 0,9 | 82,37 | 5 / 0 |
| | 2b | 388,0 | 503,9 | 0,9 | 77,00 | 15 / 0 |
| 1337 | 2a | 409,3 | 500,2 | 0,8 | 81,83 | 5 / 0 |
| | 2b | 387,1 | 498,5 | 1,0 | 77,65 | 15 / 0 |
| 1505 | 2a | 401,1 | 500,0 | 0,9 | 80,22 | 5 / 0 |
| | 2b | 371,0 | 497,9 | 0,8 | 74,51 | 15 / 0 |
| 1649 | 2a | 398,5 | 500,0 | 0,9 | 79,70 | 5 / 0 |
| | 2b | 362,0 | 494,3 | 0,7 | 73,23 | 15 / 0 |
| 1697 | 2a | 453,6 | 500,0 | 0,9 | 90,72 | 5 / 0 |
| | 2b | 388,4 | 500,0 | 0,4 | 77,68 | 5 / 0 |
| 1745 | 2a | 449,6 | 496,9 | 0,9 | 90,48 | 5 / 0 |
| | 2b | 386,7 | 502,4 | 0,4 | 76,97 | 5 / 0 |
| Analysen:<br>Bestimmung der Aminzahl (AZ): | | | | | | |

Eine gewogene Probe des Polyetheramins wird mit Methanol verdünnt und mit HCl 1 N titriert.

Die Aminzahl (AZ) berechnet sich nach der Formel

$$(\text{Verbrauch HCl 1 N [ml]} \cdot 56{,}1\ \text{[mg/ml]})\ /\ \text{Einwaage [g]} = \text{Aminzahl [mg KOH/g]}$$

Bestimmung der Acetylierungszahl (AC):

Eine gewogene Probe des Polyetheramins wird mit einem gewogenen Überschuss an Acetylierungsgemisch (Pyridin, Essigsäureanhydrid, Eisessig) versetzt und zwei Stunden bei 110 °C gerührt. Anschließend wird mit Wasser versetzt und weitere 10 min gerührt. Nach dem Abkühlen wird mit Natronlauge 0,5 N titriert. Eine Blindprobe (nur Acetylierungsgemisch, ohne PEA-Probe) wird analog behandelt.

Die AC berechnet sich nach der Formel

(Verbrauch NaOH 0,5 N [ml] für Blindwert - Verbrauch NaOH 0,5 N [ml] für Probe) • 56,1 [mg/ml]) • 0,5/Einwaage [g] = Acetylierungszahl [mg KOH/g]

Bestimmung der tertiären Aminzahl (tert. AZ):

Eine gewogene Probe des Polyetheramins wird mit einem Überschuss an Essigsäureanhydrid behandelt, um die primären und sekundären Aminfunktionen zu maskieren. Anschließend wird mit Perchlorsäure 0,1 N titriert.

Die tert. AZ berechnet sich nach der Formel

(Verbrauch Perchlorsäure 0,1 N [mg] • 5,61 [mg/ml])/Einwaage [g] = tert. Aminzahl [mg KOH/g]

Der Aminierungsgrad ist der Quotient aus AZ und AC und ist in Prozent angegeben.

Der Kaliumionengehalt im Polyetheralkohol und im Polyetheramin wurde mit der Methode der Atomemissionsspektrometrie mit induktiv gekoppeltem Plasma bestimmt. Als Gerät wurde ein 720 ES der Firma Varian verwendet. Die Probe wurde vor der Messung mit Säure vorbehandelt.

Ergebnisse:

Unter identischen Reaktionsbedingungen wurden zu Versuchsbeginn in den beiden parallel betriebenen Rohrreaktoren an dem Katalysator A derselben Katalysatorcharge innerhalb der Messungenauigkeit zunächst gleiche Aminzahlen erhalten. Der einzige Unterschied zwischen den beiden Versuchen war die Konzentration an Kaliumionen im Polyetheralkohol-Zulauf (Beispiel 2a: 5 ppm, Beispiel 2b: 10-15 ppm). Die Aktivität der Katalysatoren in den beiden Reaktoren war zu Versuchsbeginn gleich. Im weiteren Versuchsverlauf nahm die Aktivität beider Katalysatoren ab (geringere Aminzahl und geringerer Aminierungsgrad), allerdings fiel die Aktivität des Katalysators aus Beispiel 2b stärker ab, in dessen Zulauf die höhere Kaliumionen-Konzentration vorhanden war. Im Austrag beider Reaktoren waren keine Kaliumionen messbar, d.h. die Kaliumionen verblieben auf dem Aminierungskatalysator. Als einziger Unterschied zwischen den beiden Versuchen war die Ablagerung von Kaliumionen auf dem Katalysator offensichtlich ursächlich für die Aktivitätseinbußen. Durch Temperaturerhöhung um 10 °C auf 203 °C nach einer Versuchsdauer von 1169 h konnte in beiden Versuchen der Aminierungsgrad bzw. die Aminzahl gesteigert werden, allerdings war die Aktivität der beiden unterschiedlich mit Kaliumionen belasteten Katalysatoren auch bei dieser höheren Temperatur (203 °C) unterschiedlich.

Durch eine Spülung des Katalysators mit z.B. Wasser/Ammoniak nach einer Laufzeit von 1649 h konnte die Katalysatoraktivität in Versuch 2a, bei dem als Feed Pluriol® P230 mit einem Kaliumionengehalt von 5 ppm eingesetzt worden war, fast vollständig wiederhergestellt werden (Aminzahl und Aminierungsgrad erreichen fast die Anfangswerte des Versuchs), während bei Versuch 2b, bei dem Pluriol® P230 mit einem Kaliumionengehalt von 10 - 15 ppm ppm eingesetzt worden war, die Katalysatoraktivität nicht den ursprünglichen Zustand erreichte (Aminzahl und Aminierungsgrad erreichen bei weitem nicht die Anfangswerte des Versuchs), weil die Spüldauer dazu noch nicht lang genug war.

3. Herstellung von Pluriol® P230 mit einem Kaliumionengehalt von 5 ppm bzw. 10-15 ppm

Zu einer Mischung aus Monopropylenglykol und Kaliumhydroxid werden ca. 2,5 Mol-Äquivalente Propylenoxid dosiert und die Mischung fünf Stunden lang bei 130 - 140 °C gerührt. Nach Abkühlung wird Phosphorsäure zugesetzt, bis ein pH-Wert von 7 erreicht ist. Der resultierende Niederschlag wird abfiltriert. Je nach Güte der Fällung und der Filtration werden in verschiedenen Chargen Kaliumionen-Restgehalte von 5 bzw. von 10-15 Gew.-ppm im Poly-etheralkohol analysiert.

**Patentansprüche**

1. Verfahren zur Herstellung eines Polyetheramins durch Umsetzung eines Polyetheralkohols, der zuvor in Gegenwart einer basischen Kaliumverbindung als Katalysator synthetisiert wurde, mit Ammoniak in Gegenwart von Wasserstoff und eines Katalysators in einem Reaktor oder mehreren Reaktoren, **dadurch gekennzeichnet, dass** der eingesetzte Polyetheralkohol einen Gehalt an Kaliumionen von < 10 Gew.-ppm aufweist, wobei als Polyetheralkohole Diole der allgemeinen Formeln II

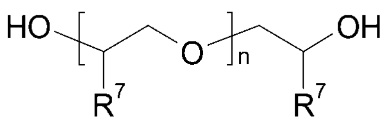

(II)

eingesetzt werden, n jeweils eine ganze Zahl zwischen 1 und 50 und $R^7$ lineares $C_1$- Alkyl bedeuten.

2. Verfahren nach einem der beiden vorhergehenden Ansprüche, **dadurch gekennzeichnet, dass** man die Umsetzung des Polyetheralkohols zum Polyetheramin in der Flüssigphase bei einem Absolutdruck im Bereich von 50 bis 220 bar durchführt.

3. Verfahren nach einem der vorhergehenden Ansprüche, **dadurch gekennzeichnet, dass** man die Umsetzung des Polyetheralkohols zum Polyetheramin bei einer Temperatur im Bereich von 150 bis 240 °C durchführt.

4. Verfahren nach einem der vorhergehenden Ansprüche, **dadurch gekennzeichnet, dass** man die Umsetzung des Polyetheralkohols zum Polyetheramin unter Einsatz von Ammoniak in einem Molverhältnis, pro Mol alkoholischer Hydroxylgruppe im Polyetheralkohol, im Bereich von 1,5 bis 500 durchführt.

5. Verfahren nach einem der vorhergehenden Ansprüche, **dadurch gekennzeichnet, dass** bei der Umsetzung des Polyetheralkohols zum Polyetheramin der Katalysator im Reaktor bzw. in den Reaktoren als Festbett angeordnet ist.

6. Verfahren nach einem der vorhergehenden Ansprüche, **dadurch gekennzeichnet, dass** es kontinuierlich durchgeführt wird.

7. Verfahren nach einem der beiden vorhergehenden Ansprüche, **dadurch gekennzeichnet, dass** die Umsetzung des Polyetheralkohols zum Polyetheramin in einem oder mehreren Rohrreaktor/en oder Rohrbündelreaktor/en erfolgt.

8. Verfahren nach einem der drei vorhergehenden Ansprüche, **dadurch gekennzeichnet, dass** die Umsetzung bei einer Frischgasfahrweise oder einer Kreisgasfahrweise erfolgt.

9. Verfahren nach dem vorhergehenden Anspruch, **dadurch gekennzeichnet, dass** man die Umsetzung bei einem Kreisgasmengenstrom im Bereich von 50 bis 1000 Normkubikmeter Kreisgas / ($m^3_{Kat.}$ • h) bzw. einem Frischgasmengenstrom im Bereich von 1 bis 200 Normkubikmeter Frischgas / ($m^3_{Kat.}$ • h) durchführt.

10. Verfahren nach einem der vorhergehenden Ansprüche, **dadurch gekennzeichnet, dass** man die Umsetzung des Polyetheralkohols zum Polyetheramin bei einer Katalysatorbelastung im Bereich von 0,01 bis 10 kg Polyetheralkohol / ($l_{Kat.}$ • h) durchführt.

11. Verfahren nach einem der vorhergehenden Ansprüche, **dadurch gekennzeichnet, dass** es sich bei der basischen Kaliumverbindung um Kaliumhydroxid handelt.

12. Verfahren nach einem der vorhergehenden Ansprüche zur Herstellung von Polyetheramin der folgenden Formel durch Umsetzung von Polyetheralkohol der Formel IIa mit Ammoniak:

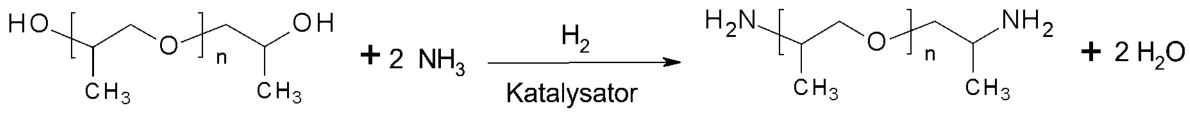


IIa

wobei Polyetheralkohol und Polyetheramin jeweils als Mischung aus Molekülen vorliegen, bei denen n im Mittel im Bereich von 2,3 bis 3,0 liegt und die Molmasse des Polyetheramins im Mittel im Bereich von 210 bis 250 g/mol liegt.

13. Verfahren nach einem der vorhergehenden Ansprüche 1 bis 11 zur Herstellung von Polyetheramin der folgenden Formel durch Umsetzung von Polyetheralkohol der Formel IIb mit Ammoniak:

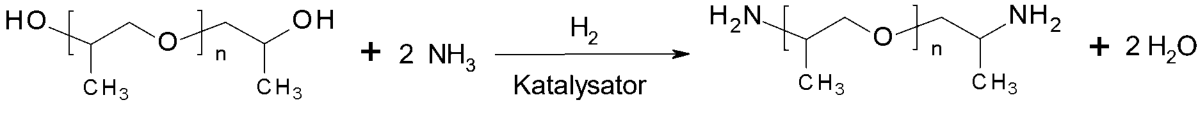


IIb

wobei Polyetheralkohol und Polyetheramin jeweils als Mischung aus Molekülen vorliegen, bei denen n im Mittel im Bereich von 31,5 bis 35,0 liegt und die Molmasse des Polyetheramins im Mittel im Bereich von 1900 bis 2100 g/mol liegt.

14. Verfahren nach einem der vorhergehenden Ansprüche, **dadurch gekennzeichnet, dass** der Katalysator für die Umsetzung des Polyetheralkohols zum Polyetheramin Kupfer und/oder Kobalt und/oder Nickel enthält.

15. Verfahren nach einem der vorhergehenden Ansprüche, **dadurch gekennzeichnet, dass** für die Umsetzung des

Polyetheralkohols zum Polyetheramin die katalytisch aktive Masse des Katalysators vor dessen Reduktion mit Wasserstoff sauerstoffhaltige Verbindungen des Aluminiums und/oder Zirkoniums und/oder Chroms und sauerstoffhaltige Verbindungen des Kupfers enthält.

16. Verfahren nach einem der vorhergehenden Ansprüche, **dadurch gekennzeichnet, dass** für die Umsetzung des Polyetheralkohols zum Polyetheramin die katalytisch aktive Masse des Katalysators vor dessen Reduktion mit Wasserstoff sauerstoffhaltige Verbindungen des Aluminiums und/oder Zirkoniums und/oder Chroms und sauerstoffhaltige Verbindungen des Kupfers und Nickels enthält.

17. Verfahren nach einem der vorhergehenden Ansprüche, **dadurch gekennzeichnet, dass** für die Umsetzung des Polyetheralkohols zum Polyetheramin die katalytisch aktive Masse des Katalysators vor dessen Reduktion mit Wasserstoff sauerstoffhaltige Verbindungen des Aluminiums und/oder Zirkoniums und sauerstoffhaltige Verbindungen des Kupfers und Kobalts und Nickels enthält.

18. Verfahren nach einem der vorhergehenden Ansprüche, **dadurch gekennzeichnet, dass** für die Umsetzung des Polyetheralkohols zum Polyetheramin die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält.

19. Verfahren nach einem der vorhergehenden Ansprüche, **dadurch gekennzeichnet, dass** für die Umsetzung des Polyetheralkohols zum Polyetheramin die katalytisch aktive Masse des Katalysators vor dessen Reduktion mit Wasserstoff im Bereich von

15 bis 80 Gew.-% sauerstoffhaltige Verbindungen des Aluminiums, berechnet als $Al_2O_3$,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts , berechnet als CoO, und
0,2 bis 5,0 Gew.-% sauerstoffhaltige Verbindungen des Zinns, berechnet als SnO, enthält.

20. Verfahren nach einem der vorhergehenden Ansprüche 1 bis 16, **dadurch gekennzeichnet, dass** für die Umsetzung des Polyetheralkohols zum Polyetheramin die katalytisch aktive Masse des Katalysators vor dessen Reduktion mit Wasserstoff im Bereich von 20 bis 85 Gew.-% sauerstoffhaltige Verbindungen des Zirkoniums, berechnet als $ZrO_2$,

1 bis 30 Gew.-% sauerstoffhaltige Verbindungen des Kupfers, berechnet als CuO,
14 bis 70 Gew.-% sauerstoffhaltige Verbindungen des Nickels, berechnet als NiO, und
0 bis 5 Gew.-% sauerstoffhaltige Verbindungen des Molybdäns, berechnet als $MoO_3$, enthält.

21. Verfahren nach einem der vorhergehenden Ansprüche 1 bis 15, **dadurch gekennzeichnet, dass** für die Umsetzung des Polyetheralkohols zum Polyetheramin es sich bei dem Katalysator um einen Kobalt-haltigen Katalysator enthaltend Mangan und Phosphor handelt.

22. Verfahren nach einem der vorhergehenden Ansprüche 1 bis 15, **dadurch gekennzeichnet, dass** für die Umsetzung des Polyetheralkohols zum Polyetheramin es sich bei dem Katalysator um einen Kobaltschwamm-Katalysator oder um einen Nickelschwamm-Katalysator handelt.

23. Verfahren nach einem der vorhergehenden Ansprüche, **dadurch gekennzeichnet, dass** aus dem Reaktionsprodukt der Umsetzung durch Destillation

(i) zunächst ggf. unumgesetzter Ammoniak über Kopf abgetrennt wird,
(ii) Wasser über Kopf abgetrennt wird,
(iii) ggf. vorhandene Nebenprodukte mit einem niedrigeren Siedepunkt als dem des Verfahrensprodukts über Kopf, ggf. zusammen mit noch vorhandenem Wasser, abgetrennt werden,
(iv) das Verfahrensprodukt Polyetheramin über Sumpf abgetrennt wird.

## Claims

1. A process for producing a polyetheramine by reacting a polyether alcohol, which was previously synthesized in the

presence of a basic potassium compound as catalyst, with ammonia in the presence of hydrogen and a catalyst in one or more reactors, **characterized in that** the polyether alcohol used has a potassium ion content of < 10 wt.-ppm, wherein polyether alcohols used are diols of the general formula II

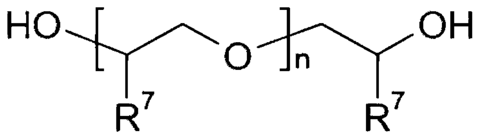

(II)

n is an integer from 1 to 50, and R7 represents linear C1-alkyl.

2. The process according to any of the preceding claims, **characterized in that** the conversion of the polyether alcohol to the polyetheramine is carried out in the liquid phase at an absolute pressure in the range of 50 to 220 bar.

3. The process according to any of the preceding claims, **characterized in that** the conversion of the polyether alcohol to the polyetheramine is carried out at a temperature in the range of 150 to 240°C.

4. The process according to any of the preceding claims, **characterized in that** the conversion is carried out using ammonia in a molar ratio, per mole of alcoholic hydroxyl group in the polyether alcohol, in the range of 1.5 to 500.

5. The process according to any of the preceding claims, **characterized in that** the catalyst is arranged as a fixed bed in the reactor or reactors.

6. The process according to any of the preceding claims, **characterized in that** it is carried out continuously.

7. The process according to any of the preceding claims, **characterized in that** the conversion takes place in one or more tubular reactors or tube bundle reactors.

8. The process according to any of the preceding claims, **characterized in that** the conversion is carried out with fresh gas operation or cycle gas operation.

9. The process according to Claim 8, **characterized in that** the conversion is carried out at a cycle gas flow rate in the range of 50 to 1000 $Nm^3$ of cycle gas/($m^3$ catalyst · h) or a fresh gas flow rate in the range of 1 to 200 $Nm^3$ of fresh gas/($m^3$ catalyst · h).

10. The process according to any of the preceding claims, **characterized in that** the conversion is carried out at a catalyst loading in the range of 0.01 to 10 kg polyether alcohol/(L catalyst · h).

11. The process according to any of the preceding claims, **characterized in that** the basic potassium compound is potassium hydroxide.

12. The process according to any of Claims 1 to 11 for producing a polyetheramine of the following formula by reacting a polyether alcohol of formula IIa with ammonia:

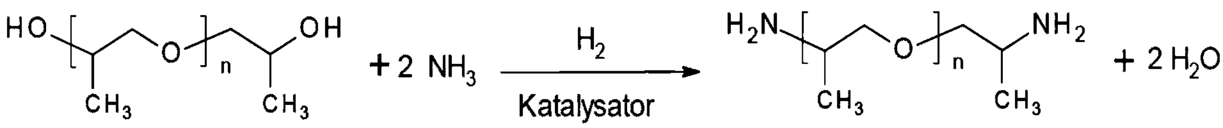


IIa

where polyether alcohol and polyetheramine are each present as a mixture of molecules in which n on average lies in the range of 2.3 to 3.0, and the molecular weight of the polyetheramine on average lies in the range of 210 to 250 g/mol.

**13.** The process according to any of Claims 1 to 11 for producing a polyetheramine of the following formula by reacting a polyether alcohol of formula IIb with ammonia:

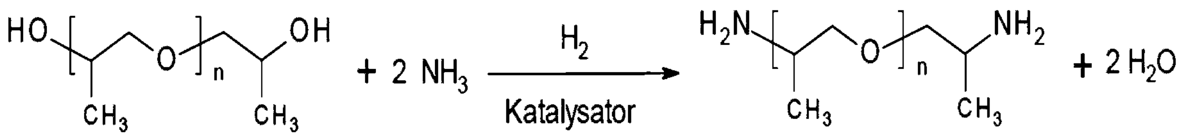


IIb

where polyether alcohol and polyetheramine are each present as a mixture of molecules in which n on average lies in the range of 31.5 to 35.0, and the molecular weight of the polyetheramine on average lies in the range of 1900 to 2100 g/mol.

**14.** The process according to any of the preceding claims, **characterized in that** the catalyst for the conversion contains copper and/or cobalt and/or nickel.

**15.** The process according to any of the preceding claims, **characterized in that** the catalytically active mass of the catalyst before its reduction with hydrogen contains oxygen-containing compounds of aluminum and/or zirconium and/or chromium, and oxygen-containing compounds of copper.

**16.** The process according to any of the preceding claims, **characterized in that** the catalytically active mass before reduction contains oxygen-containing compounds of aluminum and/or zirconium and/or chromium, and oxygen-containing compounds of copper and nickel.

**17.** The process according to any of the preceding claims, **characterized in that** the catalytically active mass before reduction contains oxygen-containing compounds of aluminum and/or zirconium, and oxygen-containing compounds of copper, cobalt, and nickel.

**18.** The process according to any of the preceding claims, **characterized in that** the catalytically active mass before reduction contains oxygen-containing compounds of aluminum, copper, nickel, and cobalt, and 0.2 to 5.0 wt.-% oxygen-containing tin compounds, calculated as SnO.

**19.** The process according to any of the preceding claims, **characterized in that** before reduction the catalytically active mass contains:

- 15 to 80 wt.-% oxygen-containing compounds of aluminum (as $Al_2O_3$),
- 1 to 20 wt.-% oxygen-containing compounds of copper (as CuO),
- 5 to 35 wt.-% oxygen-containing compounds of nickel (as NiO),
- 5 to 35 wt.-% oxygen-containing compounds of cobalt (as CoO), and
- 0.2 to 5.0 wt.-% oxygen-containing compounds of tin (as SnO).

**20.** The process according to any of Claims 1 to 16, **characterized in that** before reduction the catalytically active mass contains:

- 20 to 85 wt.-% oxygen-containing compounds of zirconium (as $ZrO_2$),
- 1 to 30 wt.-% oxygen-containing compounds of copper (as CuO),
- 14 to 70 wt.-% oxygen-containing compounds of nickel (as NiO), and
- 0 to 5 wt.-% oxygen-containing molybdenum compounds (as $MoO_3$).

**21.** The process according to any of Claims 1 to 15, **characterized in that** the catalyst is a cobalt-containing catalyst including manganese and phosphorus.

**22.** The process according to any of Claims 1 to 15, **characterized in that** the catalyst is a cobalt sponge catalyst or nickel sponge catalyst.

**23.** The process according to any of the preceding claims, **characterized in that** from the reaction product, by distillation:

(i) any unreacted ammonia is first removed overhead,
(ii) water is removed overhead,
(iii) any by-products with a lower boiling point than the product are removed overhead optionally together with remaining water), and
(iv) the polyetheramine product is removed from the bottom.

## Revendications

1. Procédé de fabrication d'une polyétheramine par conversion d'un polyétheralcool, préalablement synthétisé en présence d'un composé basique du potassium comme catalyseur, avec de l'ammoniac en présence d'hydrogène et d'un catalyseur dans un ou plusieurs réacteurs, **caractérisé en ce que** le polyétheralcool utilisé présente une teneur en ions potassium inférieure à 10 ppm en poids, les polyétheralcools employés étant des diols de la formule générale II :

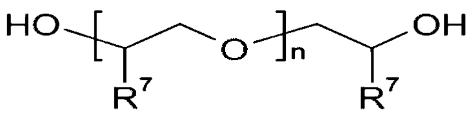

(II)

n est un nombre entier compris entre 1 et 50 et R7 représente un groupe alkyle linéaire C1.

2. Procédé selon l'une des revendications précédentes, **caractérisé en ce que** la conversion du polyétheralcool en polyétheramine est réalisée en phase liquide à une pression absolue comprise entre 50 et 220 bar.

3. Procédé selon l'une des revendications précédentes, **caractérisé en ce que** la conversion du polyétheralcool en polyétheramine est réalisée à une température comprise entre 150 et 240 °C.

4. Procédé selon l'une des revendications précédentes, **caractérisé en ce que** la conversion du polyétheralcool en polyétheramine est réalisée avec de l'ammoniac dans un rapport molaire, par mole de groupe hydroxyle alcoolique dans le polyétheralcool, compris entre 1,5 et 500.

5. Procédé selon l'une des revendications précédentes, **caractérisé en ce que** le catalyseur utilisé pour la conversion du polyétheralcool en polyétheramine est disposé sous forme de lit fixe dans le ou les réacteurs.

6. Procédé selon l'une des revendications précédentes, **caractérisé en ce qu'**il est réalisé de manière continue.

7. Procédé selon l'une des revendications précédentes, **caractérisé en ce que** la conversion du polyétheralcool en polyétheramine est réalisée dans un ou plusieurs réacteurs tubulaires ou faisceaux de tubes.

8. Procédé selon l'une des revendications précédentes, **caractérisé en ce que** la conversion est réalisée selon un mode de fonctionnement en gaz frais ou en gaz recyclé.

9. Procédé selon la revendication précédente, **caractérisé en ce que** la conversion est réalisée avec un débit de gaz recyclé compris entre 50 et 1000 $Nm^3/(m^3 \cdot h)$ de catalyseur ou un débit de gaz frais compris entre 1 et 200 $Nm^3/(m^3 \cdot h)$ de catalyseur.

10. Procédé selon l'une des revendications précédentes, **caractérisé en ce que** la conversion est réalisée avec une charge catalytique comprise entre 0,01 et 10 kg de polyétheralcool/(L de catalyseur·h).

11. Procédé selon l'une des revendications précédentes, **caractérisé en ce que** le composé basique du potassium est de l'hydroxyde de potassium.

12. Procédé selon l'une des revendications 1 à 11 pour la fabrication d'une polyétheramine de la formule suivante par conversion d'un polyétheralcool de formule IIa avec de l'ammoniac:

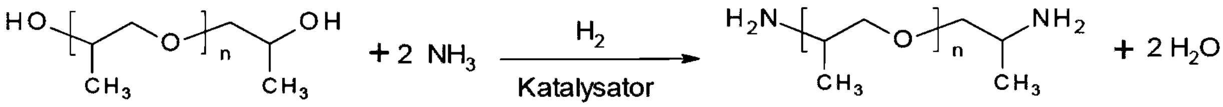


IIa

le polyétheralcool et la polyétheramine étant chacun un mélange de molécules pour lesquelles n a en moyenne une valeur comprise entre 2,3 et 3,0, et la masse molaire de la polyétheramine étant en moyenne comprise entre 210 et 250 g/mol.

**13.** Procédé selon l'une des revendications 1 à 11 pour la fabrication d'une polyétheramine de la formule suivante par conversion d'un polyétheralcool de formule IIb avec de l'ammoniac:

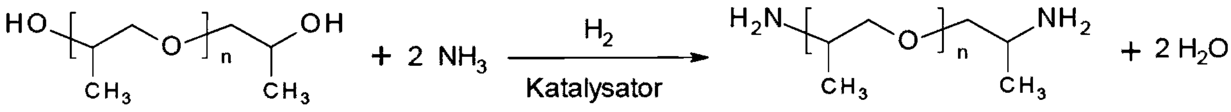


IIb

le polyétheralcool et la polyétheramine étant chacun un mélange de molécules pour lesquelles n a en moyenne une valeur comprise entre 31,5 et 35,0, et la masse molaire de la polyétheramine étant en moyenne comprise entre 1900 et 2100 g/mol.

**14.** Procédé selon l'une des revendications précédentes, **caractérisé en ce que** le catalyseur utilisé pour la conversion du polyétheralcool en polyétheramine contient du cuivre et/ou du cobalt et/ou du nickel.

**15.** Procédé selon l'une des revendications précédentes, **caractérisé en ce que** la masse catalytiquement active du catalyseur avant réduction à l'hydrogène contient des composés oxygénés de l'aluminium et/ou du zirconium et/ou du chrome et des composés oxygénés du cuivre.

**16.** Procédé selon l'une des revendications précédentes, **caractérisé en ce que** la masse catalytiquement active contient des composés oxygénés de l'aluminium et/ou du zirconium et/ou du chrome ainsi que des composés oxygénés du cuivre et du nickel.

**17.** Procédé selon l'une des revendications précédentes, **caractérisé en ce que** la masse catalytiquement active contient des composés oxygénés de l'aluminium et/ou du zirconium ainsi que des composés oxygénés du cuivre, du cobalt et du nickel.

**18.** Procédé selon l'une des revendications précédentes, **caractérisé en ce que** la masse catalytiquement active contient des composés oxygénés de l'aluminium, du cuivre, du nickel et du cobalt ainsi que de 0,2 à 5,0 % en poids de composés oxygénés de l'étain, calculés en tant que SnO.

**19.** Procédé selon l'une des revendications précédentes, **caractérisé en ce que** la masse catalytiquement active contient 15 à 80 % en poids de composés oxygénés de l'aluminium (exprimés en $Al_2O_3$), 1 à 20 % en poids de composés oxygénés du cuivre (exprimés en CuO), 5 à 35 % en poids de composés oxygénés du nickel (exprimés en NiO), 5 a 35 % en poids de composés oxygénés du cobalt (exprimés en CoO), et 0,2 à 5,0 % en poids de composés oxygénés de l'étain (exprimés en SnO).

**20.** Procédé selon l'une des revendications 1 à 16, **caractérisé en ce que** la masse catalytiquement active contient 20 à 85 % en poids de composés oxygénés du zirconium (exprimés en $ZrO_2$), 1 à 30 % en poids de composés oxygénés du cuivre (exprimés en CuO), 14 à 70 % en poids de composés oxygénés du nickel (exprimés en NiO), et 0 à 5 % en poids de composés oxygénés du molybdène (exprimés en $MoO_3$).

**21.** Procédé selon l'une des revendications 1 à 15, **caractérisé en ce que** le catalyseur est un catalyseur à base de cobalt contenant du manganèse et du phosphore.

**22.** Procédé selon l'une des revendications 1 à 15, **caractérisé en ce que** le catalyseur est un catalyseur de type éponge

de cobalt ou de type éponge de nickel.

**23.** Procédé selon l'une des revendications précédentes, **caractérisé en ce que**, dans le produit réactionnel de la conversion

(i) l'ammoniac éventuellement non converti est séparé en tête par distillation,
(ii) l'eau est séparée en tête,
(iii) les sous-produits éventuels présentant un point d'ébullition inférieur à celui du produit du procédé sont séparés en tête, éventuellement avec de l'eau résiduelle, et
(iv) la polyétheramine, produit du procédé, est séparée en fond.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2011067199 A1
- EP 696572 A1
- WO 09092724 A1
- EP 1028138 A2
- WO 07096317 A1
- US 3580952 A
- JP 49014158 B
- JP 49014159 B
- WO 2011087793 A1
- WO 2014009292 A1
- EP 2014059181 W
- EP 2014059145 W
- EP 636409 A1
- EP 742045 A1

### In der Beschreibung aufgeführte Nicht-Patentliteratur

- Fixed-Bed Reactors. Ullmann's Encyclopedia of Industrial Chemistry, vol. B 4, 199-238